# EUROPEAN PATENT SPECIFICATION

(11) **EP 1 711 052 B1**
(45) Date of publication and mention of the grant of the patent: **09.05.2012**
(21) Application number: 05706932.0
(22) Date of filing: 20.01.2005
(51) Int. Cl.: A01K 67/027, C12N 15/85, A61K 49/00, C07K 14/82, C12N 5/10, C12N 15/90

(54) **MOUSE MODEL FOR PSORIASIS AND PSORIATIC ARTHRITIS**
MAUSMODELL FÜR PSORIASIS UND ARTHRITIS PSORIATICA
SOURIS UTILISÉES COMME MODÈLE POUR PSORIASIS CHEZ L'HOMME

(30) Priority: 21.01.2004 EP 04001181
(43) Date of publication of application: 18.10.2006
(73) Proprietor: Boehringer Ingelheim International GmbH, 55216 Ingelheim (DE)
(72) Inventor: WAGNER, Erwin, A-1030 WIEN (AT); ZENZ, Rainer, A-8046 GRAZ (AT)
(74) Representative: Hammann, Heinz
(86) International application number: PCT/EP2005/000517
(87) International publication number: WO 2005/070199

(56) References cited:
- WO-A-96/27019
- WO-A-02/062135
- SCHOEN MICHAEL P: "Animal models of psoriasis - What can we learn from them?" JOURNAL OF INVESTIGATIVE DERMATOLOGY, vol. 112, no. 4, April 1999 (1999-04), pages 405-410, XP002287489 ISSN: 0022-202X cited in the application
- ZENZ RAINER ET AL: "c-Jun regulates eyelid closure and skin tumor development through EGFR signaling." DEVELOPMENTAL CELL, vol. 4, no. 6, June 2003 (2003-06), pages 879-889, XP008032625 ISSN: 1534-5807 cited in the application
- LI GUOCHUN ET AL: "c-Jun is essential for organization of the epidermal leading edge." DEVELOPMENTAL CELL, vol. 4, no. 6, June 2003 (2003-06), pages 865-877, XP008032624 ISSN: 1534-5807 cited in the application
- SZABOWSKI AXEL ET AL: "c-Jun and junB antagonistically control cytokine-regulated mesenchymal-epidermal interaction in skin" CELL, vol. 103, no. 5, 22 November 2000 (2000-11-22), pages 745-755, XP002287490 ISSN: 0092-8674 cited in the application
- ANGEL PETER ET AL: "Function and regulation of AP-1 subunits in skin physiology and pathology" ONCOGENE, vol. 20, no. 19 Review Issue 2, 30 April 2001 (2001-04-30), pages 2413-2423, XP002287505 ISSN: 0950-9232
- WERNER S ET AL: "PARACRINE REGULATION OF KERATINOCYTE PROLIFERATION AND DIFFERENTIATION" TRENDS IN CELL BIOLOGY, ELSEVIER SCIENCE LTD, XX, vol. 11, no. 4, April 2001 (2001-04), pages 143-146, XP001154567 ISSN: 0962-8924

## Description

The invention relates to the field of psoriasis.

Psoriasis, affecting about 2% of the population, is one of the most common human skin disorders that affect skin and joints. It is characterized by complex alterations of various cell types. This includes epidermal hyperproliferation and altered differentiation, as well as angiogenesis and dilation of dermal blood vessels (Schön, 1999; Lebwohl, 2003). In addition, a mixed leukocytic infiltrate is seen. It is composed of activated T lymphocytes, neutrophiles within the dermis and epidermal microabscesses, lining macrophages, and an increased number of dermal mast cells. Cytokines including tumor necrosis factor-α (TNF-α), and interleukin-1 (IL-1), interferon-γ (INF-γ), IL-6, IL-8, vascular endothelial growth factor (VEGF) and transforming growth factor-α (TGF-α) are thought to mediate the psoriatic tissue alterations (Schön, 1999).

For decades the ongoing controversy is whether psoriasis results from primary abnormalities in the epidermis or is immunologically based (Nickoloff *et al.,* 2000). Although evidence is accumulating that it has an immunological basis others interpret psoriasis as a genetically determined, abnormal epithelial response pattern to infection and/or physicochemical skin insults.

It has become clear that psoriatic skin is a hotbed of epidermal growth factors and inflammatory mediators. Supportive evidence of a key role for such mediators comes from patients who respond to immunsuppressive, anti-inflammatory, and antiproliferative therapies such as cyclosporine, methotrexate, tacrolimus, corticosteroids, and ultraviolet-light-activated psoralen. However, extensive efforts aimed at transgenically delivered inflammatory mediators or keratinocyte growth factors to the skin have not completely reproduced the psoriatic phenotype (Xia *et al.,* 2003), which has thus far only been faithfully modeled in animals by transplanting psoriatic skin onto mice with severe combined immunodeficiency disease (SCID). So far no reported mouse model for psoriasis mimics all characteristics seen in psoriasis in humans, including psoriatic arthritis which is present in up to 40% of psoriasis patients.

Since there is no naturally occurring animal skin disease mirroring both phenotype and immunopathogenesis of psoriasis, research into the pathogenesis of this common skin disorder has been severely hampered.

Consequently, there is a need for an efficient and significant animal model for the study of psoriasis and for testing drug candidates effective in the treatment of this disorder.

It was an object of the invention to provide an animal model for psoriasis, including psoriatic arthritis.

The solution of the problem underlying the invention is based on the molecular mechanisms associated with the transcription factor AP-1.

The transcription factor AP-1 is generated by a series of dimers of products of the ***Fos, Jun**,* and CREB/ATF protein families (Eferl and Wagner, 2003), as well as other bZip proteins. In addition, associations have been observed between Fos or Jun and the p65 subunit of NFκB (Stein *et al.,* 1993), and ATF-2 and p50- NFκB (Du *et al.,* 1993). Combinatorial association can draw on three ***Jun*** genes (*c-**J**un, **J**unB, **J**unD*), four ***Fos*** genes (*c-**F**os, **F**osB, **F**ra-1, **F**ra-2*) and several CREB/ATF genes. Despite the high degree of homology in the overall structural features, the different members of the Fos, Jun and CREB families exhibit significant differences, which lead to subtle differences in DNA binding and transcriptional activation (Angel and Herrlich, 1994) suggesting specific functions in gene regulation for individual dimers. The members of the AP-1 family are engaged in the control of cell proliferation as well as various types of differentiation, and also in neural function and stress responses. AP-1 is one of the key factors that translate external stimuli both into short- and long-term changes of gene expression.

The transcription factor ***c-Jun*** interacts with related and unrelated transcription factors, gaining influence on different and seemingly unrelated signal pathways controlling diverse target genes. ***c-Jun*** receives regulatory input originating outside of the cell, traversing the plasma membrane, cytoplasm, and nuclear envelope in a cascade of biochemical reactions (Herrlich and Ponta, 1989; Ransone and Verma, 1990; Karin and Smeal, 1992). These signals can modify the transcription of the ***c-Jun*** gene or affect the activity of the ***c-Jun*** protein post-translationally. The regulation of ***c-Jun's*** transactivation potential takes place at two different levels: increased phosporylation in the transactivation domain causes enhanced transcriptional activity, and enhanced binding to DNA is due to dephosphorylation in the DNA binding region (Sachsenmaier and Radler-Pohl, 1994). Unstimulated mammalian cells contain low, but detectable, amounts of ***c-Jun*** protein. In this state ***c-Jun*** is phosphorylated constitutively at serines and threonines close to its C-terminal DNA binding domain. Phosporylation in this region markedly reduces DNA binding and transactivation ability of ***c-Jun** in vitro* (Boyle *et al.,* 1991) and *in vivo* (Lin *et al.,* 1992). In contrast to the negative effect of hyperphosphorylation in DNA binding, enhanced phosphorylation at the N-terminus is required for the activation of the transactivation function of c-Jun. Serines 73 and 63 are located close to a stretch of amino acids described as the "proline-rich region", which is required for transactivation and can serve as *in vitro* substrates for mitogen-activated protein (MAP) kinases (Pulverer *et al.,* 1991). Transcriptional regulation by c-Jun is highly cell type dependent (Imler *et al.,* 1988; Bohmann and Tjian, 1989; Baichwald and Tjian, 1990), because of different upstream activators, partner molecules, or downstream targets present in the different cell types. A functional role for ***c-Jun*** in the skin has been suggested for differentiation and carcinogenesis. Since c-***Jun*** knock-out mice are not viable (Johnson *et al.,* 1993; Hilberg *et al.,* 1993), the consequence of lacking ***c-Jun*** in skin development and skin carcinogenesis could only be investigated by generation of conditional *c-**J**un* knock-out mice (*c-**J**un* ^{*Δ*ep}) (Zenz R. *et al.,* 2003; Li *et al.,* 2003). Mice lacking *c-**J**un* in keratinocytes (*c-**J**un* ^{Δep}) develop normal skin but express reduced levels of EGFR in the eyelids, leading to open eyes at birth, as observed in EGFR null mice. Primary keratinocytes from *c-**J**un* ^{Δep} mice proliferate poorly, show increased differentiation, and form prominent cortical actin bundles, most likely because of decreased expression of EGFR and its ligand HB-EGF. In the absence of ***c-Jun,*** tumor-prone K5-SOS-F transgenic mice develop smaller papillomas, with reduced expression of EGFR in basal keratinocytes. Thus, using three experimental systems, we showed that EGFR and HB-EGF are regulated by ***c-Jun**,* which controls eyelid development, keratinocyte proliferation, and skin tumor formation.

All the ***Jun*** proteins (***c-Jun, JunB*** and ***JunD**)* are similar with respect to their primary structure and their DNA-binding specificity. However, ***JunB,*** due to a small number of amino acid changes in its basic-leucine zipper region exhibits a 10-fold weaker DNA-binding activity and a decreased homodimerization property compared to ***c-Jun.*** Transfection studies have suggested that ***J**unB* is a repressor of AP-1 mediated transactivation and transformation most likely due to the preferential formation of inactive ***c-Jun*/*JunB*** dimers (Chiu *et al.,* 1989; Deng and Karin, 1993). However, ***in vitro*** and ***in vivo*** data have demonstrated that ***JunB*** can also be a strong transactivator depending on both the interacting partner and the promoter context (Chiu *et al.,* 1989; Li *et al.,* 1999).

Since ***J**unB* knock-out mice are not viable (Schorpp-Kistner *et al*., 1999), the consequence of lacking ***JunB*** in skin development and skin carcinogenesis can only be investigated by generation of conditional ***J**unB* knock-out mice (***J**unB*^{Δep}).

Depending on the experimental system, ***c-Jun*** and ***JunB*** *h*ave been thought to have opposite functions in regulating gene expression, thereby influencing the biological output of various external and internal signals triggering AP-1 activity. To verify this, in the experiments leading to the invention, a novel experimental system was used by generating **keratinocyte**-specific double knock-out mice that are deficient for both ***c-Jun*** and ***JunB** (**c-Jun***^{Δep}, ***JunB** B^{Δep}*). Unexpectedly, these mice die shortly after birth preventing the analysis of phenotypes at later stages in the development of these mice.

Therefore, in the experiments describing the invention, an inducible conditional knock-out mouse that is deficient for ***c-Jun*** and ***JunB*** in the **keratinocytes** was generated.

In a first aspect, the invention relates to a mouse which is deficient for *c-Jun* and *JunB* in keratinocytes due to deletion of the C-Jun and JunB genes in keratinocytes.

In a further aspect, the invention relates to a transgenic mouse, in which deletion of the C-Jun and JunB genes has been induced in keratinocytes by expression of an inducible recombination enzyme in keratinocytes.

For clarity, the terminology and abbreviations used herein have the following meanings:
***c-Jun***^{f/f}; ***JunB***^{f/f} or ***c-Jun***^{f/f}*, **JunB***^{f/f}*,* respectively, refers to mice with a floxed ***c-Jun*** or junB locus or mice with floxed ***c-Jun*** and ***JunB*** loci, respectively.
***c-Jun***^{f/f} K5-Cre-ER (or ***JunB***^{f/f} K5-Cre-ER or ***c-Jun***^{**f**/f}*,* ***JunB***^{**f**/f} K5-Cre-ER, respectively) refers to the floxed mice, in which the respective gene(s) are not (yet) deleted, since Cre needs to be activated first. Activation of Cre, and consequently deletion of the gene(s), is achieved by applying tamoxifen. (***c-Jun***^{f/f}*, **JunB***^{f/f} K5-Cre-ER mice are representative for the above-defined mice, in which deletion of *c-Jun* and *JunB* can be specifically induced in **keratinocytes**).
***c-Jun***^{f/f} K5-Cre (or junB^{f/f} K5-Cre or ***c-Jun***^{f/f}, ***JunB***^{f/f} K5-Cre, respectively), refers to mice in which the respective gene(s) have been deleted in **keratinocytes** (Cre is in the active status). ***c-Jun*** ^{f/f}, ***JunB***^{f/f} K5-Cre mice are representative for the above-defined mice, which are deficient for *c-Jun* and *JunB* in the keratinocytes of the epidermis (such mice are also designated *"c-**J**un* ^{*Δ*ep***}, ***J**unB* ^{Δep*} mice").

The constituents K5, Cre and ER can be replaced by any other suitable keratinocyte -specific promoter, recombination enzyme or gene encoding a biological molecule, respectively, which, upon binding of its ligand, induces activation of the recombination enzyme, e.g. another hormone receptor like the progesterone receptor binding domain, which is activated upon binding of an antiprogestin, e.g. RU486 binding (Minamino *et al.,* 2001).

In a further aspect, the invention relates to methods for generating c-***J**un*^{Δep*}, ***J**unB*^{Δep*} mice, as defined in the claims.

The principle of suitable methods is based on known protocols for generating transgenic mice, preferably on embryonic stem (ES) cell technology. The essential features of suitable preferred methods for obtaining the *c-**J**un*^{Δep*}*, **J**unB*^{Δep*} mice of the invention are, on the one hand, that the ***c-Jun*** and ***JunB*** genes are flanked with recognition sites for a site specific recombination enzyme (recombinase), and that, on the other hand, the recombinase has been provided by crossing the conditional knock-out mouse with a transgenic mouse expressing an inducible recombinase in keratinocytes. keratinocyte specific expression can be achieved by using a promoter specific keratinocytes; examples for suitable promoters are K5, K6, K10 or K 14 (Jiang *et al.,* 1991).

In the experiments describing the invention, the inducible gene deletion system enabling to delete both genes in adult mice, as described by Vasioukhin et al., 1999), was used. This system allows to prevent postnatal lethality of ***J**un*^{Δep*}, *J**u**nB*^{Δep} mice, which would not allow for analysis of phenotypes at later stages in the development. This system uses a K5-Cre-ER transgenic mouse line, which expresses a Cre-recombinase estrogene receptor fusion under the control of the keratinocyte-specific Keratin 5 promoter (K5) specifically in the **keratinocytes*.*** Using this system, both genes in adult mice can be deleted by consecutive intraperitoneal tamoxifen injection (Brocard *et al.,* 1999; Vasioukhin *et al.,* 1999). In keratinocytes expressing the Cre-ER fusion protein tamoxifen, an anti-estrogene, binds to the mutated estrogen receptor domain. This mutated fusion protein does not bind the endogenous 17β-estradiol, whereas it binds the synthetic ligand tamoxifen (Danielian *et al.,* 1993). Tamoxifen binding is inducing a conformational change in the protein structure leading to an active Cre-recombinase. The active Cre-recombinase is transported into the nucleus and deletes the DNA-sequences between 2 loxP sites (floxed gene).

For obtaining the **keratinocyte** -specific double knock-out mice of the invention, which are deficient for both *c-jun* and *junB* mice, according to a preferred embodiment, the inducible loxP/Cre system is used. To date, this system is considered to be the most reliable experimental setup for spatio-temporally controlled site-specific somatic gene deletion in vivo. The deletion of the gene(s) of interest (in the case of the present invention ***c-Jun*** and ***JunB***) can be induced either by intraperitoneal injection or topic application of an anti-estrogen like tamoxifen or OH-tamoxifen to the skin (Vasioukhin *et al.,* 1999).

Alternatively to the loxP/Cre-system, other spatio-temporally controlled site-specific somatic gene deletion systems can be used to generate keratinocyte-specific double knock-out mice for ***c-Jun*** and ***JunB**.*

Examples for such alternative methods for engineering the conditional knock-out mice of the invention are the Flp-FRT and the phiC31-att site-specific recombinase systems. As the loxP/Cre-system, these systems fulfill the requirements of having the gene(s) of interest flanked with recognition sites for the site specific recombination enzyme and of providing the recombination enzyme by crossing the conditional knock-out mouse with a transgenic mouse expressing a constitutively active or inducible recombinase in the tissue of interest (Branda and Dymecki, 2004).

Alternatively to spatio-temporally controlled site-specific somatic gene deletion systems, siRNA techniques are described for skin-specific gene silencing.

Recently, plasmid-based systems using RNA-polymerase III promoters to drive short hairpin RNA (shRNA) molecules have been established to produce siRNA and the generation of knockdown ES cell lines with transgenic shRNA was reported (Kunath *et al.,* 2003). Furthermore, viral-mediated delivery for specific silencing of target genes in liver and brain in mice through expression of small interfering RNA (siRNA) has been established (Xia *et al.,* 2002). These systems can be adapted to generate knockdown mice or knocking down ***c-Jun*** and ***JunB*** in the **keratinocytes** of mice, e.g. by designing siRNA constructs that are expressed in the **keratinocytes** of mice after delivery by means of suitable gene delivery protocols.

In a further aspect, the invention relates to keratinocytes deficient for *c-Jun* and *JunB*, derived from a mouse in which the *c-Jun* and *JunB* genes have been specifically deleted in the **keratinocytes** (c-Jun^{Δep*}, JunB^{Δep*}). Keratinocytes can be obtained according to known methods, e.g. by using a protocol as described by or by Carroll *et al.,* 1995, Carroll and Molès, 2000, or the following protocol (described in US patent 6,566,136):
Sections of skin obtained from sacrificed mice that have been shaved and washed are separated in dermis and epidermis, e-g. by treatment with trypsin, for example by flotation of the samples of skin tissue in a trypsin solution (for example about 0.5%) during a time sufficient to provoke cell separation. The dermis is separated and the epidermis is subsequently placed in a medium for achieving a suspension. The medium for achieving a suspension may contain a solution of soy trypsin inhibitor (SBTI) and is put in contact with the cells for a time sufficient to inactivate the trypsin and to provoke the release of the cells. The cells are grown in a serum-free tissue culture medium and filtered in order to obtain the desired keratinocytes.

The resulting primary keratinocytes are subsequently seeded, in a suitable cell concentration, e.g. about 1.2x10⁴ cells/cm², on previously coated culture plates. The culture plates are usually coated with a composition that increases fixation and growth of keratinocytes, usually a solution containing fibronectin and of collagen type I.

It was surprisingly found in the experiments describing the invention that mice, after induced **keratinocyte** -specific deletion of ***c-Jun*** and ***JunB*** (*c-**J**un*^{Δep*}, ***J**unB*^{Δep*} mice), develop a psoriasis-like disease reflecting the hallmarks of psoriasis in humans within 2 weeks after tamoxifen injection. This phenotype was only seen after deletion of both ***c-Jun*** and ***JunB**,* but not after **keratinocyte** -specific deletion of ***c-Jun*** and ***JunB*** alone.

Thus the invention relates, in a further aspect, to the use of *c-**J**un*^{Δep*}, ***J**unB*^{Δep*} mice as defined in the claims as an animal model for psoriasis and psoriatic arthritis.

The present invention provides a unique mouse model recapitulating many of the histological and molecular characteristics seen in the skin and joints of psoriatic patients. The phenotype develops very rapidly with 100% efficiency. The disease is not characterized by a generalized inflammatory response, but is prominent to hairless areas of the skin (ears, paws and tail) reminiscent of the symmetrically distributed psoriatic lesions in humans which are well demarcated from adjacent symptom-less skin. The diseased **keratinocytes** mirrors the changes in the cytokine and chemokine network reported for psoriasis. Expression profiling revealed further evidence that the changes in gene expression resemble most of the documented genetic changes described for human psoriasis.

The *c-**J**un*^{Δep*}, ***J**unB*^{Δep*} mice of the invention and c-Jun and JunB deficient keratinocytes derived therefrom are useful for testing drugs against psoriasis and psoriatic arthritis *in vivo.*

For example, given the role for pro-inflammatory cytokines such as IL-1, IL-8 and TNF-α in the pathogenesis of psoriasis, the mice of the invention may be utilized to evaluate specific inhibitors of these cytokine pathways or to test compounds whether they have an inhibitory effect. A specific illustration is the evaluation of inhibitors of the TNF-α pathway. For instance, ***c-Jun***^{f/f}*, **JunB***^{f/f} K5-Cre-ER mice are treated with tamoxifen to obtain *c-**J**un* ^{Δep*}*, **J**unB* ^{Δep*} mice that develop the psoriasis-like disease. Upon first observation of symptoms, e.g. skin abnormality, the mice receive an inhibitor of this cytokine, e.g. a monoclonal antibody against mouse TNF-α, a well documented inhibitor of this cytokine *in vivo.* The mice are then **to** be observed for the progression of skin lesions and the extent of disease progression can be compared to placebo-treated animals. Novel inhibitors of the TNF-α pathway, such as decoy receptors, TNF-synthesis inhibitors, MAP kinase inhibitors or therapeutic siRNA can also be used and the effects of treatment can be compared to anti-TNF-α treated animals. Likewise, test compounds, e.g. from compound libraries, can be applied to the animals and the effect of the compounds evaluated. The extent of lesion development post-treatment as well as the histopathologic makeup of the lesion can be analyzed (Carroll *et al.,* 1995). Also the effect of compounds on disease induction can be determined by treating ***c-Jun***^{f/f}*, **JunB***^{f/f} K5-Cre-ER mice prior to tamoxifen administration. Changes in systemic markers of inflammation, such as cytokine levels, acute phase proteins, lymphocyte subsets and immunoglobulin levels and isotypes can be evaluated. The extent of inflammation pre- and post-treatment may also be assessed through determining the vascular permeability of diseased skin using extravasation markers such as Evans blue dye or ¹²⁵I labeled albumin.

The mouse model of the invention is not limited to the evaluation of inhibitors of pro-inflammatory cytokines. For instance, novel inhibitors of leukocyte trafficking, such as adhesion molecules or chemokines, can be tested in the model similar to the aforementioned protocol. Also, specific inhibitors of the immune system such as inhibitors of T-cell activation, cytokine production or co-stimulation can be used. Agents or methods aimed at the induction of T regulatory cells can also be assessed.

Finally, the mouse model of the invention is useful to test the effect of modulators directed specifically against keratinocyte function or other cells of the skin. Such agents can be evaluated both *in vivo* and *in vitro.* For instance, mice can be induced for disease as described above and keratinocyte / skin specific agents applied via topical or systemic routes. The effect of such agents, or test compounds, on lesion development can be measured via histopathologic analysis (Carroll *et al.,* 1995) and compared to untreated mice. Likewise, primary keratinocyte cultures or keratinocyte lines can be established in vitro from diseased animals using standard protocols (Carroll *et al.,* 1995). Test compounds can be administered to the cultured cells in various doses and the effect on keratinocyte proliferation, viability, morphology and cytokine / growth factor production can be evaluated. Furthermore, cultured double knock-out keratinocytes, which can be obtained from the mice of the invention according to standard protocols, e.g. as described by Carroll *et al.* 1995; Carroll and Molès, 2000; Zenz *et al.,* 2003, or *in vitro* skin tissue equivalents (Szabowski et al., 2000) or three-dimensional skin culture models (Carroll and Molès, 2000) can be used to screen for drugs inhibiting the production of keratinocytes-derived growth factors and cytokines responsible for psoriasis.

### Brief description of the Figures:

Figure 1
   A.) Schematic outline of the floxed *c-**J**un* and floxed ***J**unB* locus and the inducible Cre-ER transgene used to delete both genes upon tamoxifen application.
   B.) Treatment scheme. Adult mice were injected intraperitoneally 5 times with 1mg tamoxifen and analyzed 14 days thereafter.
   C.) Southern Blot for *c-**J**un* and ***J**unB* to confirm deletion of both genes after tamoxifen application specifically in the **keratinocytes** of adult mice.
   D.) RPA (RNAse protection assay) for AP-1 genes after deletion of *c-Jun* and *JunB* demonstrating downregulation of all AP-1 genes beside fra-2.
Figure 2
   A.) Macroscopy of ear, feet and tail from adult mice after inducible deletion of *c-**J**un* and ***J**unB* in the **keratinocytes** of adult mice resembling psoriasis.
   B.) Histology of psoriatic mouse ears reflecting the hallmarks of psoriasis: abnormally thickened epidermis, parakeratosis (nucleated keratinocytes in the cornified layer), thickened keratinized upper layer (hyperkeratosis), and fingerlike epidermal projections into the dermis (rete ridges). Epidermal microabscesses and the typical inflammatory cell infiltrate are seen: intraepidermal T-cells (CD3 staining), increased numbers of neutrophils in the epidermis (esterase staining), and macrophages in the dermis (esterase- and F4/80-staining).
   C.) H&E staining from affected mouse finger demonstrating granulocytic infiltrates into the joint region similar to psoriatic arthritis.

In the Examples, the following materials and methods were used:

### Generation of JunB^{f/}^{f} mice

A floxed and frt-flanked neomycin resistance and thymidine kinase gene selection cassette was inserted into a SmaI site present in the 5' untranslated region (UTR) of *junB.* The 3' loxP site was inserted into the XhoI site, 161 bp downstream of the translation stop codon.

A diphtheria toxin gene was included for selection against random integrants. The linearized targeting construct was electroporated into HM-1 ES cells and the identification of homologous recombinants by PCR using two sets of primers was performed as described previously (Schorpp-Kistner *et al.,* 1999). The neomycin and thymidine kinase genes were deleted by transient transfection of a vector expressing flp recombinase. Two ES cell clones carrying a floxed allele of *JunB* were injected into C57BL/6 blastocysts and several chimeras from one ES cell clone transmitted the mutant allele to their offspring.

### Generation of c-Jun^{Δep*}, JunB^{Δep*} mice

Mice carrying a floxed c-jun allele (***c-Jun***^{f/f}*,* Behrens *at al.,* 2002) were crossed to mice carrying a floxed ***JunB*** allele (***JunB***^{f/f}, Kenner *et al.,* 2004) to obtain mice double-floxed mice (***c-Jun***^{f/f}, ***JunB***^{f/f}). Double floxed mice were crossed to transgenic mice expressing the Cre recombinase-estrogene receptor fusion under the control of the keratinocyte-specific Keratin 5 promoter (K5-Cre-ER; Brocard *et al.,* 1997) to obtain ***c-Jun***^{f/f}, ***JunB***^{f/f} and ***c-Jun***^{f/f}, ***Jun**B*^{f/f}; K5-Cre-ER mice.

*Inducible deletion of **c-Jun***^{f/f}, ***JunB***^{f/f /f}

8 weeks old experimental mice ***c-Jun***^{f/f}*, **JunB***^{f/f}; K5-Cre-ER) and control mice (c-jun^{f/f}; junB^{f/f}) were injected intraperitoneally 5 times with 1mg tamoxifen (Sigma; Vasioukhin *et al.,* 1999).

### Southern Blot and RNase protection assay (RPA)

For the ***c-Jun*** Southern blot, 10µg of epidermal DNA was digested with XbaI (for c-jun) yielding a 6.9 kb fragment for the floxed ***c-Jun*** allele and a 3.3 kb fragment for the deleted c-jun allele (Behrens *et al.,* 2002). For the junB Southern 10µg of genomic DNA was digested with PstI yielding a 2.3 kb fragment for the floxed ***JunB*** allele and a 0.7 kb fragment for the deleted ***J**unB* allele. For detection of the bands a 176 bp PstI/HindIII fragment of ***J**unB* was used as probe (Kenner *et al.,* 2004). For the RNase protection assay total epidermal RNA was isolated with the TRIZOL protocol (Sigma). RNase protection assays were performed using the RiboQuant multi-probe RNase protection assay systems mJun/Fos (PharMingen) according to the manufacturer's protocol.

### Histology

Tissues were fixed overnight with neutral buffered 4% PFA at 4°C and either directly or after decalcification (bone) in 0.5% EDTA for 12 days embedded in paraffin. Five-micrometer sections were stained either with hematoxylin and eosin (H&E) or processed further. Immunohistochemical staining for anti CD3 and F4/80 (Santa Cruz) was performed after antigen-retrieval (Dako S1699) with the MultiLink Dako system (Dako E0453) according to the manufacturer's recommendations.

### Example 1

### Generation of c-Jun^{Δep*}, JunB^{Δep*} mice

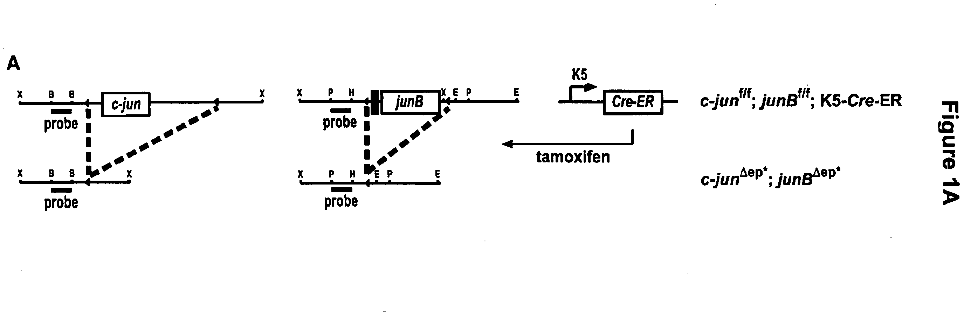

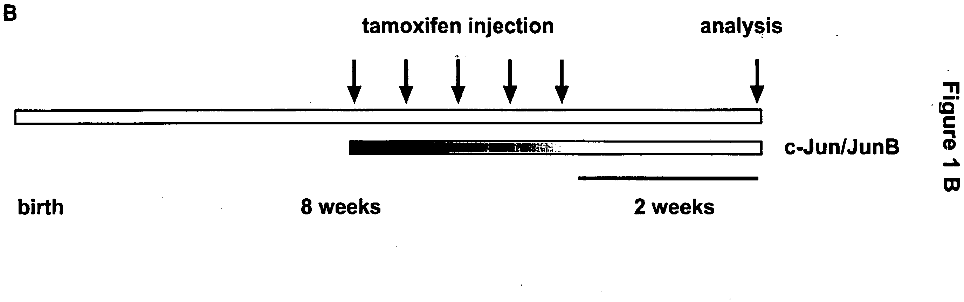

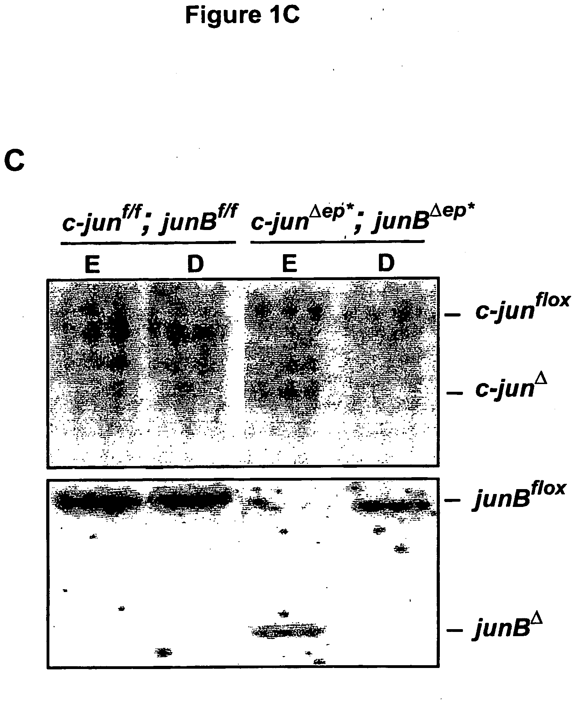

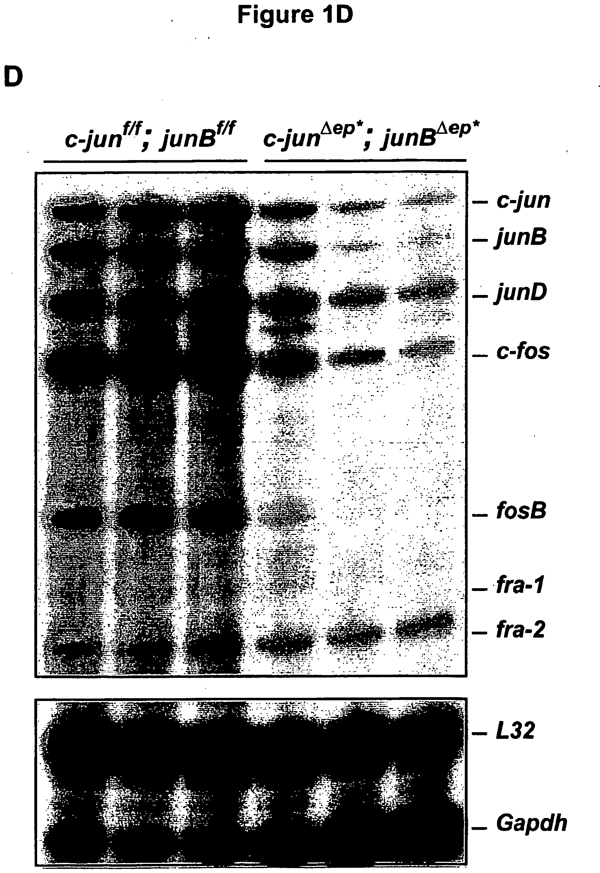

*c-Jun* ^{f/f}, *JunB* ^{f/f} mice were crossed to K5-Cre-ER transgenic mice and heterozygous progenies were intercrossed to get c-jun^{f/f}; junB^{f/f}; K5-Cre-ER and ***c-Jun***^{f/f}*, **JunB***^{f/f} mice. The approach to delete c-jun and junB in the epidermis is outlined in Fig. 1A and 1B. In brief, 8 weeks old experimental mice (***c-Jun*** ^{f/f}, ***JunB*** ^{f/f}; K5-Cre-ER) and control mice (***c-Jun***^{**f**/f}*, **JunB***^{f/f}) were injected intraperitoneally 5 times with 1 mg tamoxifen. 2 weeks after the last injection the mice were analyzed. The deletion of *c-Jun* and *JunB* was analyzed by Southern blot and RPA (RNAse protection assay). Southern blot analyses for *c-**Jun*** and ***JunB*** deletion (Fig. 1C) showed deletion in both cases. The remaining signal for floxed c-jun and floxed junB is explained by incomplete deletion and the inflammatory infiltrate seen in the **keratinocytes** of *c-**J**un*^{Δep*}*, J**u**nB*^{Δep*} mice (Fig. B). Quantification of AP-1 mRNA in the **keratinocytes** of *c-**Jun***^{Δep*}*, **JunB***^{Δep*} mice showed significant down regulation of ***c-Jun*** and ***JunB*** mRNA and also for other AP-1 genes except fra2.

### Example 2

### Characterization of c-Jun^{Δep*}, JunB^{Δep*} mice

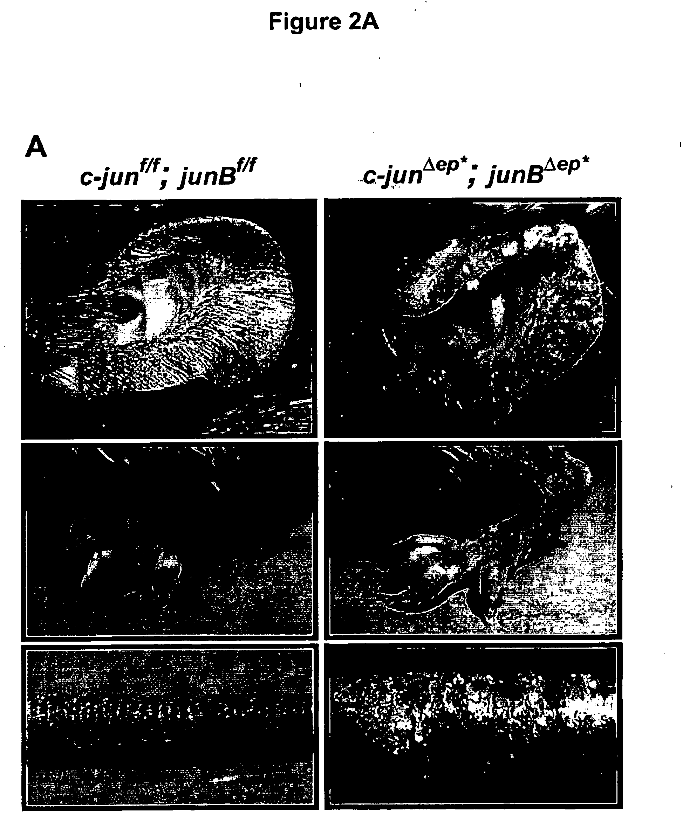

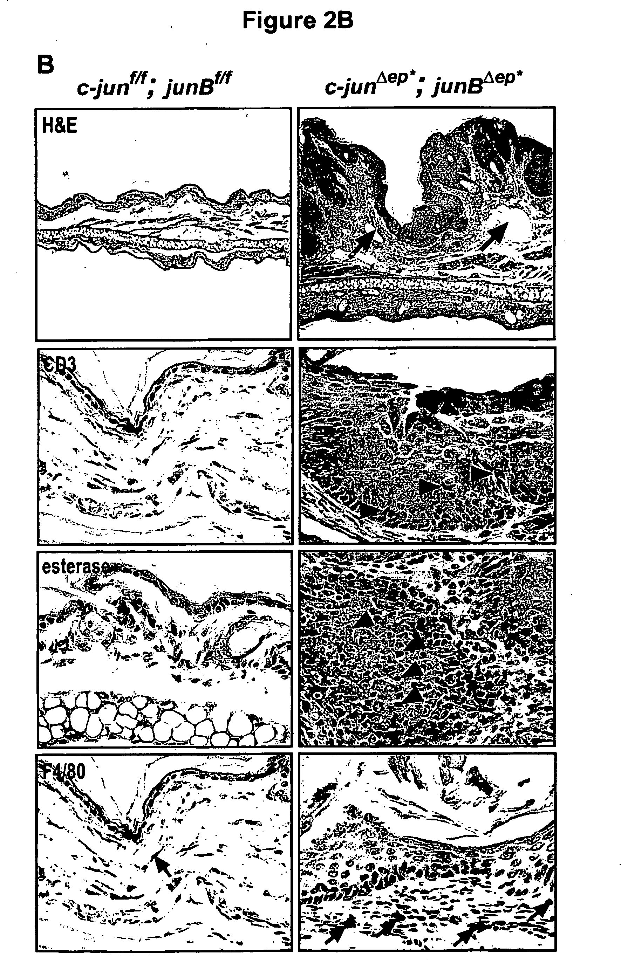

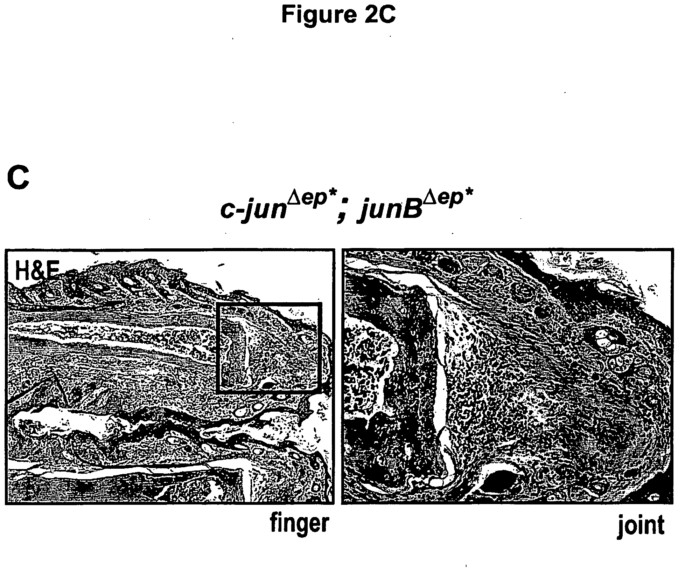

Mice obtained according to the treatment described in Example 1 develop a phenotype resembling psoriasis within 2 weeks. Hairless skin like ear, tail and feet were dramatically affected (Fig. 2A). Histological examination (Fig. 2B) revealed abnormally thickened epidermis, parakeratosis (nucleated keratinocytes in the cornified layer), thickened keratinized upper layer (hyperkeratosis), and fingerlike epidermal projections into the dermis (rete ridges). Furthermore, epidermal microabscesses and the typical inflammatory cell infiltrate are seen: intraepidermal T-cells (CD3 staining), increased numbers of neutrophils in the epidermis (esterase staining), and macrophages in the dermis (esterase- and F4/80-staining). H&E staining from affected mouse finger demonstrating granulocytic infiltrates into the joint region resembling psoriatic arthritis (Fig. 2C).

### References

Baichwal V.R. and Tjian R. (1990). Control of c-Jun activity by interaction of a cell-specific inhibitor with regulatory domain d: Differences between v- and c-Jun. Cell 63, 815-25.
Behrens A., Sibilia M., David JP., Mohle-Steinlein U., Tronche F., Schutz G., Wagner EF. (2002). Impaired postnatal hepatocyte proliferation and liver regeneration in mice lacking c-jun in the liver. EMBO J. 21, 1782-90.
Bohmann D. and Tjian R. (1989). Biochemical analysis of transcriptional activation by Jun: Differential activity of c- and v-Jun. Cell 59, 709-17.
Boyle W.J., Smeal T., Defize L.H.K., Angel P., Woodgett J.R., Karin M., and Hunter T. (1991). Activation of protein kinase C decreases phosphorylation of c-Jun at sites that negatively regulate its DNA-binding activity. Cell 64, 573-584.
Branda C.S. and Dymecki S.M. (2004). Talking about a Revolution. The impact of site-specific recombinases on genetic analyses in Mice. Dev.Cell 6(1):7-28.
Brocard J., Warot X., Wendling O., Messaddeq N., Vonesch JL., Chambon P., Metzger D. (1997). Spatio-temporally controlled site-specific somatic mutagenesis in the mouse. Proc. Natl. Acad. Sci. 94, 14559-63.
Carroll JM., Luetteke NC., Lee DC., Watt FM. (1995). Suprabasal integrin expression in the epidermis of transgenic mice results in developmental defects and a phenotype resembling psoriasis. Cell 83:957-968.
Carroll JM, Molès JP. (2000)A three-dimensional skin culture model for mouse keratinocytes: application to transgenic mouse keratinocytes. Exp Dermatol 2000: 9: 20-24.
Chiu R., Angel P., Karin M. (1989). Jun-B differs in its biological properties from, and is a negative regulator of, c-Jun. Cell 59, 979-86.
Danielian PS., White R., Hoare SA., Fawell SE., Parker MG. (1993). Identification of residues in the estrogene receptor that confer differential sensitivity to estrogen and hydroxytamoxifen. Mol Endocrinol 7(2):232-40.
Deng T., Karin M. (1993). JunB differs from c-Jun in its DNA-binding and dimerization domains, and represses c-Jun by formation of inactive heterodimers. Genes Dev. 7(3):479-90.
Du W., Thanos D., and Maniatis T. (1993). Mechanisms of transcriptional synergism between distinct virus-inducible enhancer elements. Cell 74, 887-98.
Efer1, R. and Wagner, E.F. (2003). AP-1: A double-edged sword in tumorigeneis. Nature Reviews Cancer 3, 859-868.
Herrlich P. and Ponta H. (1989). "Nuclear" oncogenes convert extracellular stimuli into changes in the genetic program. Trends Genet. 5: 112-5.
Imler J.L., Ugarte E., Wasylyk C. and Wasylyk B. (1988) v-jun is a transcriptional activator, but not in all cell lines. Nucleic Acids Res. 16, 3005-12.
Hilberg F., Agguzi A., Howells N., and Wagner E.F. (1993). c-Jun is essential for normal mouse development and hepatogenesis. Nature 365, 179-181.
Jiang CK., Epstein HS., Tomic M., Freedberg IM. and Blumenberg M. (1991). Functional comparison of the upstream regulatory DNA sequences of four human epidermal keratin genes. J. Invest. Dermatol., Vol 96, 162-167.
Johnson R.S., van Lingen B., Papaioannou V.E., and Spiegelman B.M. (1993). A null mutation at the c-jun locus causes embryonic lethality and retarded cell growth in culture. Genes Dev. 7, 1309-1317.
Karin M. and Smeal T. (1992). Control of transcription factors by signaling transduction pathways: The beginning of the end. Trends Biochem. Sci. 17, 418-22.
Kenner L, Hoebertz A, Beil T, Keon N, Karreth F, Eferl R, Scheuch H, Szremska A, Amling M, Schorpp-Kistner M, Angel P, Wagner EF. (2004): Mice lacking JunB are osteopenic due to cell-autonomous osteoblast and osteoclast defects. J Cell Biol. 164(4):613-23.
Kunath T., Gish G., Lickert H., Jones N., Pawson T., Rossant J. (2003). Transgenic RNA interference in ES cell derived embryos recapitulates a genetic null phenotype. Nat.Biotchnol. 21 (5): 559-61
Lebwohl M (2003). Psoriasis. Lancet 361(9364):1197-204
Li B., Tournier C., Davis RJ., Flavell RA. (1999). Regulation of IL-4 expression by the transcription factor JunB during T helper cell differentiation. EMBO 18(2):420-32.
Li G., Gustafson-Brown C., Hanks SK., Nason K., Arbeit JM., Pogliano K., Wisdom RM., Johnson RS. (2003). c-jun is essential for organization of the epidermal leading edge. Dev. Cell 4(6): 865-77.
Lin A., Frost J., Deng T., Smeal T., al-Alawi N., Kikkawa U., Hunter T., Brenner D., and Karin M. (1992). Casein kinase II is a negative regulator of c-Jun DNA binding and AP-1 activity. Cell. 70, 777-89.
Minamino T, Gaussin V, DeMayo FJ, Schneider MD (2001). Inducible gene targeting in postnatal myocardium by cardiac-specific expression of a hormone-activated Cre fusion protein. Circ Res. Mar 30; 88(6): 587-92
Nickoloff BJ., von den Driesch P., Raychaudhuri SP., Boehncke W-H., Morhenn VB., Farber EM., Holik MF., Schröder JM. (2000). Is psoriasis a T-cell disease? Exp Dermatol 9: 359-375.
Pulverer B.J., Kyriakis J.M., Avruch K., Nikolakaki E., and Woodgett J.R. (1991). Phosphorylation of c-jun mediated by MAP kinases. Nature 353, 670-674.
Ransone L.J. and Verma I.M. (1990). Nuclear proto-oncogenes Fos and Jun. Annu. Rev. Cell Biol. 6, 539-57.
Sachsenmaier C. and Radler-Pohl A. (1994). Regulation of c-Jun activity by phosphorylation. In: The fos and jun families of transcription factor/ edited by Peter E. Angel and Peter A. Herrlich. CRC Press. pp. 61-70.
Schön MP (1999). Animal models of psoriasis - what can we learn from them? J Invest Dermatol. 112(4):405-10
Schorpp-Kistner M., Wang ZQ., Angel P., Wagner EF. (1999). JunB is essential for mammalian placentation. EMBO J. 18(4):934-48.
Stein B., Baldwin A.S. Jr., Ballard D., Greene A, Angel P. and Herrlich P. (1993). Cross-coupling of the NFκBp65 and Fos/Jun transcription factors produces potentiated biological functions. EMBO J. 12, 3879-91.
Szabowski A., Mass-Szabowski N., Andrecht S., Kolbus A., Schorpp-Kistner M., Fusenig NE., Angel P. (2000). c-Jun and junB antagonistically control cytokine-regulated mesenchymal-epidermal interaction in skin. Cell 103(5):745-55
Vasioukhin V., Degenstein L. Wise B., Fuchs E. (1999). The magic touch: Genome targeting in epidermal stem cells induced by tamoxifen application to mouse skin. Proc. Natl. Acad. Sci. 96: 8551-8556.
Xia H., Mao Q., Paulson H.L., Davidson B.L. (2002) siRNA-mediated gene silencing in vitro and in vivo. Nat.Biotechnol. 20(10): 1006-10.
Xia YP., Li B., Hylton D., Detmar M., Yancopoulos GD., Rudge JS. (2003) Transgenic delivery of VEGF to mouse skin leads to an inflammatory condition resembling human psoriasis. Blood 102(1):161-8.
Zenz R., Scheuch H., Martin P., Frank C., Eferl R., Kenner L., Sibilia M., Wagner EF. (2003). c-Jun regulates eyelid closure and skin tumor development trough EGFR signaling. Dev. Cell 4(6): 879-89.

## Claims

1. A mouse deficient for c-Jun and JunB in keratinocytes due to deletion of the *c-Jun* and *JunB* genes in keratinocytes

2. The mouse of claim 1, in which deletion of the *c-Jun* and *JunB* genes has been induced by expression of an inducible recombination enzyme in keratinocytes.

3. The mouse of claim 1 or 2, in which deletion of the *c-Jun* and *JunB* genes in keratinocytes has been mediated by an inducible site-specific recombination enzyme (recombinase) whose synthesis was under transcriptional control of a keratinocyte-specific promoter, and which has acted on specific recognition sites flanking the *c-Jun* and *JunB* genes in the genome.

4. A method of obtaining the mouse of any of claims 1 to 3, wherein a transgenic mouse having the *c-Jun* and *JunB* genes flanked with recognition sites for an inducible site-specific recombination enzyme is crossed with a transgenic mouse expressing said inducible recombination enzyme under transcriptional control of keratinocyte-specific promoter, and wherein the recombination activity of said recombination enzyme is subsequently induced, resulting in deletion of said genes in keratinocytes.

5. The method of claim 4, wherein the inducible recombination enzyme is Cre-recombinase fused to the estrogene receptor, and wherein expression of said inducible recombination enzyme is under the transcriptional control of a keratinocyte-specific promoter.

6. The method of claim 5, wherein the recognition sites are IoxP sites and the recombinase enzyme is Cre recombinase, which is fused to the estrogen receptor, under the control of a keratinocyte specific promoter, and wherein keratinocyte-specific deletion of the *c-Jun* and *JunB* genes is induced by applying an anti-estrogen.

7. The method of claim 6, wherein the anti-estrogen is applied by intraperitoneal injection or topic application.

8. The method of claim 6 or 7, wherein the anti-estrogen is tamoxifen

9. Keratinocytes deficient for c-Jun and JunB due to deletion of the *c-Jun* and *JunB* genes, derived from the mouse of claims 1 to 3.

10. The mouse of any of claims 1 to 3 as an animal model for psoriasis and psoriatic arthritis in humans.

## Patentansprüche

1. Maus, die aufgrund der Deletion der c-Jun- und JunB-Gene in Keratinocyten c-Jun- und JunB-defizient in Keratinocyten ist.

2. Maus nach Anspruch 1, worin die Deletion der c-Jun- und JunB-Gene induziert worden ist durch Expression eines induzierbaren Rekombinationsenzyms in Keratinocyten.

3. Maus nach Anspruch 1 oder 2, worin die Deletion der c-Jun- und JunB-Gene vermittelt worden ist durch ein induzierbares, Sequenz-spezifisches Rekombinationsenzym (Rekombinase), dessen Synthese unter der transcriptionellen Kontrolle eines Keratinocyt-spezifischen Promotors erfolgte und das auf spezifische Erkennungsstellen einwirkte, welche die c-Jun- und JunB-Gene im Genom flankieren.

4. Verfahren zum Erhalten der Maus nach irgendeinem der Ansprüche 1 bis 3, worin eine transgene Maus, deren c-Jun- und JunB-Gene von Erkennungsstellen für ein induzierbares, Sequenz-spezifisches Rekombinationsenzym flankiert sind, mit einer transgenen Maus gekreuzt wird, die das induzierbare, Sequenz-spezifische Rekombinationsenzym unter der transcriptionellen Kontrolle eines Keratinocyt-spezifischen Promotors exprimiert, und worin die Rekombinationsaktivität des Rekombinationsenzyms anschließend induziert wird, wodurch die Gene in den Keratinocyten deletiert werden.

5. Verfahren nach Anspruch 4, worin das induzierbare Rekombinationsenzym Cre-Rekombinase ist, die an den Estrogen-Rezeptor fusioniert ist, und worin die Expression des induzierbaren Rekombinationsenzyms unter der transcriptionellen Kontrolle eines Keratinocyt-spezifischen Promotors erfolgt.

6. Verfahren nach Anspruch 5, worin die Erkennungstellen IoxP-Stellen sind und das Rekombinase-Enzym Cre-Rekombinase ist, welche an den Estrogen-Rezeptor fusioniert ist, unter der Kontrolle eines Keratinocyt-spezifischen Promotors, und worin die Keratinocyt-spezfifische Deletion der c-Jun- und JunB-Gene durch Anwendung eines Anti-Estrogens induziert wird.

7. Verfahren nach Anspruch 6, worin das Anti-Estrogen durch intraperitoneale Injektion oder topische Anwendung appliziert wird.

8. Verfahren nach Anspruch 6 oder 7, worin das Anti-Estrogen Tamoxifen ist.

9. Keratinocyten, die aufgrund der Deletion der c-Jun- und JunB-Gene c-Jun- und JunB-defizient sind, abgeleitet von der Maus nach den Ansprüchen 1 bis 3.

10. Maus nach irgendeinem der Ansprüche 1 bis 3 als Tiermodell für Psoriasis and psoriatische Arthritis beim Menschen.

## Revendications

1. Souris déficiente en *c-Jun* et *JunB* dans les kératinocytes en raison de la délétion des gènes *c-Jun* et *JunB* dans les kératinocytes.

2. Souris selon la revendication 1, chez laquelle la délétion des gènes *c-Jun* et *JunB* a été induite par l'expression d'une enzyme de recombinaison inductible dans des kératinocytes.

3. Souris selon la revendication 1 ou 2, chez laquelle la délétion des gènes *c-Jun* et *JunB* dans les kératinocytes a été effectuée par l'intermédiaire d'une enzyme de recombinaison (recombinase) spécifique de site inductible dont la synthèse était sous le contrôle transcriptionnel d'un promoteur spécifique des kératinocytes et qui a agi sur des sites de reconnaissance spécifiques flanquant les gènes *c-Jun* et *JunB* dans le génome.

4. Méthode d'obtention de la souris selon l'une quelconque des revendications 1 à 3, dans laquelle une souris transgénique ayant les gènes *c-Jun* et *JunB* flanqués de sites de reconnaissance pour une enzyme de recombinaison spécifique de site inductible est croisée avec une souris transgénique exprimant ladite enzyme de recombinaison inductible sous le contrôle transcriptionnel du promoteur spécifique des kératinocytes, et dans laquelle l'activité de recombinaison de ladite enzyme de recombinaison est ensuite induite, entraînant la délétion desdits gènes dans les kératinocytes.

5. Méthode selon la revendication 4, dans laquelle l'enzyme de recombinaison inductible est la recombinase Cre fusionnée au récepteur des estrogènes et dans laquelle l'expression de ladite enzyme de recombinaison inductible s'effectue sous le contrôle transcriptionnel d'un promoteur spécifique des kératinocytes.

6. Méthode selon la revendication 5, dans laquelle les sites de reconnaissance sont des sites IoxP et l'enzyme recombinase est la recombinase Cre qui est fusionnée au récepteur des oestrogènes, sous le contrôle d'un promoteur spécifique des kératinocytes, et dans laquelle la délétion spécifique des kératinocytes des gènes *c-Jun* et *JunB* est induite en appliquant un anti-oestrogène.

7. Méthode selon la revendication 6, dans laquelle l'anti-oestrogène est appliqué par injection intra-péritonéale ou application topique.

8. Méthode selon la revendication 6 ou 7, dans laquelle l'anti-oestrogène est le tamoxifène.

9. Kératinocytes déficients en *c-Jun* et *JunB* en raison de la délétion des gènes *c-Jun* et *JunB*, provenant de la souris selon les revendications 1 à 3.

10. Souris selon l'une quelconque des revendications 1 à 3 en tant que modèle animal du psoriasis et de l'arthrite psoriasique chez l'être humain.
